(19) Europäisches Patentamt
European Patent Office
Office européen des brevets

(11) Numéro de publication: **0 011 059**
**B1**

# (12) FASCICULE DE BREVET EUROPEEN

(45) Date de publication du fascicule du brevet: **31.08.83**

(21) Numéro de dépôt: **79870027.4**

(22) Date de dépôt: **23.10.79**

(51) Int. Cl.³: **C 07 D 471/20**
//(C07D471/20, 221/00, 209/00, 209/00)

## (54) Procédé de préparation de la (+,-) vincadifformine et d'autres dérivés pentacycliques apparentés.

(30) Priorité: **26.10.78 US 954741**

(43) Date de publication de la demande:
**14.05.80 Bulletin 80/10**

(45) Mention de la délivrance du brevet:
**31.08.83 Bulletin 83/35**

(84) Etats contractants désignés:
**AT BE CH DE FR GB IT LU NL SE**

(56) Documents cités:
**US - A - 4 154 943**

(73) Titulaire: **OMNICHEM Société anonyme**
**12 Avenue de Broqueville**
**B-1150 Bruxelles (BE)**

(72) Inventeur: **Kuehne, Martin Eric**
**169 South Cove Road**
**Burlington Vermont 05401 (US)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

EP 0 011 059 B1

# Procédé de préparation de la (+,–) vincadifformine et d'autres dérivés pentacycliques apparentés

La présente invention est relative à un procédé de synthèse de la (±) vincadifformine et d'autres dérivés pentacycliques apparentés.

Les composés obtenus par le procédé de l'invention répondent à la formule générale suivante:

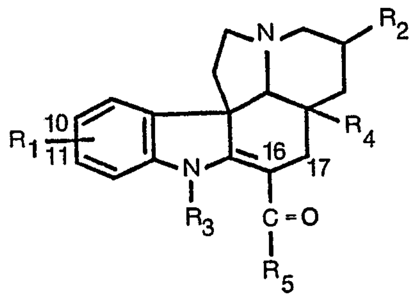

(I)

Dans cette formule $R_1$ représente un atome d'hydrogène ou d'halogène, un groupe hydroxyle ou un groupe alkyle, acyloxy ou alkoxy comportant de 1 à 4 atomes de carbone, $R_2$ représente un atome d'hydrogène ou un groupe hydroxyle, $R_3$ représente un atome d'hydrogène ou un groupe alkyle comportant de 1 à 4 atomes de carbone,

$R_4$ est un groupe alkyle, éventuellement substitué par un groupe alkoxy ou hydroxyle, comportant de 1 à 7 atomes de carbone,

$R_5$ est un groupe méthyle ou un groupe alkoxy comportant de 1 à 4 atomes de carbone.

La numérotation de la vincadifformine et de ses dérivés est celle suggérée par Le Men et Taylor (Experientia 1965, 21, 508).

La vincadifformine de formule Ia ($R_1$=$R_2$=$R_3$=H, $R_4$=éthyle, $R_5$=$OCH_3$) est un alcaloïde utilisé comme matière première pour la synthèse des alcaloïdes du groupe de la vincamine, synthèse décrite dans les brevets belges 772.005 et 848.475. La vincamine et quelques-uns de des dérivés sont des alcaloïdes bien connus utilisés en thérapeutique humaine en tant que médicament oxygénateur cérébral de grande efficacité et dont la toxicité est faible. Il a été démontré de plus, que le réarrangement de la vincadifformine en vincamine peut être appliqué à un grand nombre de dérivés de la vincadifformine pour fournir les dérivés correspondants de la vincamine (voir par exemple les brevets français 76.22336 et 76.22275 et le brevet belge 816.692).

Certains dérivés de la vincadifformine peuvent être également utilisés comme produits de départ pour la synthèse d'alcaloïdes bis-indoliques utilisés en thérapeutique anti-cancéreuse.

La vincadifformine de formule Ib ($R_1$= 11 methoxy, $R_2$=$R_3$=H, $R_4$=éthyle et $R_5$=méthoxy) peut être en effet N($\alpha$)-méthylé pour fournir un composé qui a été utilisé pour la synthèse de la partie vindoline de la vinblastine dont les propriétés anti-mitotiques sont bien connues (J. P. Kutney et al., J. Amer. Chem. Soc. *100*, 4220, 1978).

Deux synthèses totales de la vincadifformine sont décrites dans la littérature: Kutney et al. J. Amer. Chem. Soc. *90*, 3891, 1968 et J. V. Laronze et al. Tetrahedron Letters 491, 1974. Deux autres synthèses totales de la vincadifformine sont décrites par la demanderesse dans le brevet des Etats-Unis d'Amérique n' 4.154.943 et la demande U.S. n° 963.454 du 24 août 1978.

La méthoxy-11 vincadifformine de formule Ib (ervincéine) est un alcaloïde qui a été isolé de Vinca Erecta et décrit par D. A. Rakhimov, V. M. Malikov, M. R. Yagudaev et S. N. Yunusov (Khim. prir. Soedin. 226, 1970).

Le but de la présente invention est de fournir une méthode de synthèse de la vincadifformine et de dérivés polycycliques similaires avec de bons rendements, un nombre d'étapes réduit et en utilisant des réactifs accessibles et peu coûteux.

Plus particulièrement, le procédé de l'invention se caractérise par la condensation en une étape d'une $\alpha$-carboalcoxy ou d'une $\alpha$-alcanoyl-$\alpha$-méthyl-tetrahydro 1,2,3,4-$\beta$-carboline et d'un aldéhyde comportant une fonction électrophile. Cette condensation fournit le composé désiré avec un bon rendement et à partir de produits très accessibles.

Le brevet des Etats-Unis 4.154.943 susmentionné décrit une synthèse de la vincadifformine qui consiste essentiellement à condenser un aldéhyde fonctionnalisé avec une hexahydroazépino (4,5-b)indole-5,5-dicarboxylate de méthyle et de t-butyle. Ce dernier composé est obtenu par réaction d'un sel de malonate, de préférence un malonate de thallium, avec une $\beta$-carboline N-benzylée.

La présente méthode permet donc d'obtenir la vincadifformine de manière plus directe par réaction d'un aldéhyde fonctionnalisé avec une $\beta$-carboline beaucoup plus facilement accessible qu'une azépino-indole. On évite en outre une étape de protection-déprotection et l'utilisation d'un réactif couteux, dangereux et peu maniable tel un sel de thallium.

La carboline de départ utilisée est une $\alpha$-carboalkoxy ou $\alpha$-alcanoyl $\alpha$-méthyl-$\beta$-tetrahydro-$\beta$-carboline répondant à la formule générale suivante:

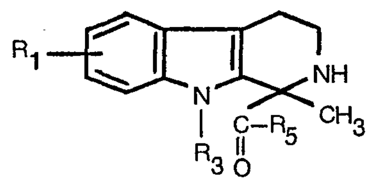

(II)

dans laquelle $R_1$, $R_3$ et $R_5$ ont les significations mentionnées pour la formule I.

Ces composés sont avantageusement obtenus par condensation de la tryptamine avec un pyruvate d'alkyle ou avec le butanedione-2,3. Le schéma suivant illustre cette réaction

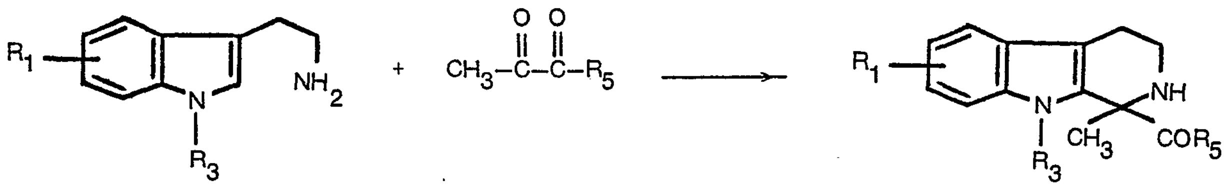

(II)

Selon la présente invention, le composé II est condensé avec un pentanal judicieusement fonctionnalisé pour fournir, en une étape, la vincadifformine ou un dérivé de la vincadifformine.

L'aldéhyde répond, par exemple, à la formule générale suivante

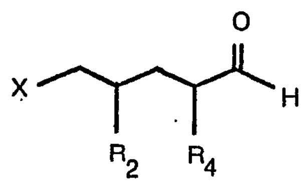

(VI)

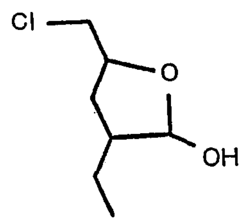

(VIa)

dans laquelle $R_2$ et $R_4$ ont les significations de la formule I, et X est un bon groupe partant.

L'aldéhyde chosi sera de préférence un alkyl-2-halogéno-5 pentanal. La fonction halogéno peut cependant être favorablement remplacée par un groupe alkylsulfonyloxy arylsulfonyloxy, ou fluoroalkylsulfonyloxy.

De manière plus générale, les aldéhydes utilisés dans le procédé de l'invention doivent pouvoir former des énamines tertiaires susceptibles d'être alkylées intramoléculairement.

A titre d'exemple d'aldéhyde répondant à cette définition, citons le chloro-5 éthyl-2 pentanal, le bromo-5 éthyl-2 pentanal, le sulfométhoxy-5 ethyl-2 pentanal.

D'autres alkyl-2 pentanals peuvent également être utilisés, ainsi le chloro-5 hydroxy-4 éthyl-2 pentanal, sous sa forme hémiacétal VIa (obtenu à partir de l'acide pentène-4-oïque correspondant par halolactonisation puis réduction au DIBAL), fournit après condensation avec la $\beta$-carboline, l'hydroxy-14 vincadifformine (I, $R_1$=$R_5$=H, $R_2$=OH, $R_4$=éthyle, $R_5$-méthoxy).

Il a été constaté en outre que, de manière générale, les aldéhydes VI peuvent être utilisés sous leur forme acétalisée.

Pour la synthèse de la vincadifformine elle-même, l'aldéhyde préféré est le chloro-5 éthyl-2 pentanal VIb.

Comme l'illustre le schéma suivant, cet aldéhyde est obtenu par condensation d'une amine primaire, par exemple, la cyclohexylamine III avec le butyraldéhyde IV pour former la butylidène-1 cyclohexylamine V.

Ce dernier dérivé fournit après réaction avec une base forte, telle que le diisopropylamide de lithium, et addition de bromo-1 chloro-3 propane, le chloro-5 pentanal VIb.

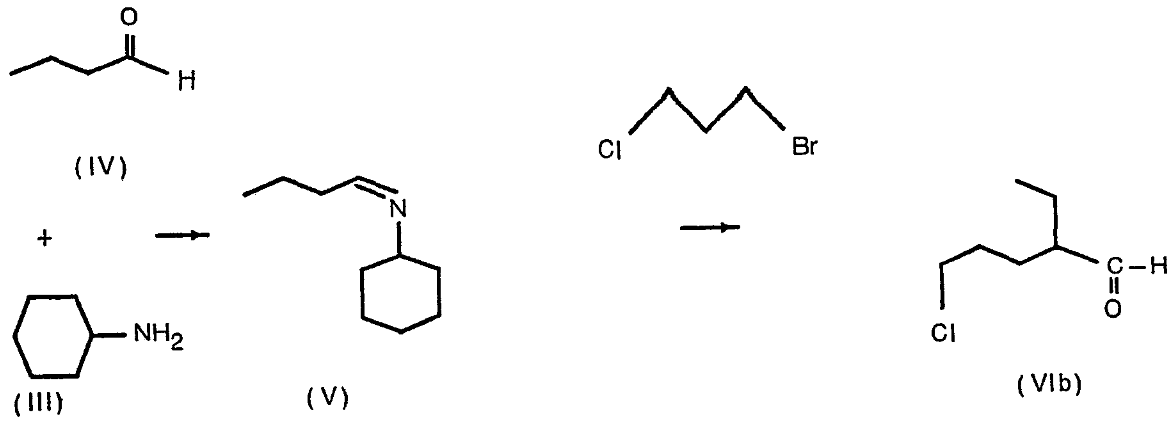

(IV) (III) (V) (VIb)

La condensation qui caractérise l'invention se produit par chauffage du composé II en présence de un à deux équivalents de l'aldéhyde VI dans un solvant adéquat et de préférence en présence d'une petite quantité de catalyseur acide. La durée de la réaction peut varier entre 10 et 160 heures.

Il est préférable d'utiliser un solvant aromatique tel que le benzène anhydre ou le toluène, mais d'autres solvants inertes dans les conditions de la réaction peuvent également être utilisés.

Il a été constaté que l'acide p-toluène sulfonique est un catalyseur particulièrement avantageux. L'eau formée lors de la formation initiale de l'énamine est éliminée du milieu réactionnel de préférence en utilisant un séparateur d'eau Dean-Stark ou un tamis moléculaire.

La température de la réaction peut varier entre 30°C et le point d'ébullition du mélange réactionnel. La température préférée se situe généralement aux environs de 100°C.

Dans quelques cas, par exemple pour la synthèse de la vincadifformine elle-même, il peut être avantageux après une période de chauffage initiale, d'ajouter une base organique telle que le diazabicycloundécène (DBU).

Les composés produits par le procédé de l'invention sont isolés en utilisant les méthodes usuelles.

Les exemples suivants illustrent le procédé de l'invention, sans toutefois la limiter:

### Exemple 1: butylidène-1 cyclohexylamine (V)

A 0°C, 16,3 g (20 ml, 0.23 mol) de butyraldéhyde sont ajoutés goutte à goutte à 21,7 g (25 ml, 0,22 mol) de cyclohexylamine (III). Après une heure d'agitation, on ajoute 6 g de sulfate de sodium anhydre. La solution est agitée durant 5 heures, puis le produit est décanté du sel hydraté. Le produit est à nouveau séché avec 10 g de sulfate de sodium anhydre, filtré puis purifié par distillation (éb. 88°C/20 mm). Le rendement est de 86%.

R.M.N. ($CDCl_3$, $\delta$): 1,0 (3H,t); 1,1—2,1 (12H); 2,3 (2H); 3,0 (1H,m); 7,8 (1H,t).

### Exemple 2: chloro-5 éthyl-2 pentanal (VIb)

A −78°C, une solution de 15,3 g (18,2 ml, 0,10 mol) de butylidene-1 cyclohexylamine (V) dans 30 ml de tétrahydrofuranne est ajoutée à une solution de diisopropylamide de lithium (0,105 mol) préparée à partir de 45,6 ml de n-butyllithium 2.3 N et 16 ml de disisopropyl amine dans 20 ml de tetrahydrofuranne sous azote.

Après 30 minutes, 11 ml (0,11 mol) de bromo-1 chloro-3 propane sont additionnés goutte à goutte en 30 minutes à −78°C.

Le milieu réactionnel est ramené à température ambiante et agité pendant 48 heures.

On ajoute alors 200 ml d'eau et on extrait deux fois avec 150 ml de chlorure de méthylène. On lave la phase organique avec 200 ml d'eau saturée en NaCl. Après concentration, on obtient une huile jaune qui est combinée à 40 g d'acide oxalique dans 350 ml d'eau. Après une distillation à la vapeur suivie par une redistillation, on obtient 5,5 g (rendement: 37%) de chloroaldéhyde (VIa).

Propriétés physico-chimiques: Eb. 43—53°C (50—120 mm Hg).

R.M.N. ($CDCl_3$, $\delta$): 9,6 (1H,d); 3,5 (2H,t); 2,2 (1H,m); 1,8 (6H,m); 0,95 (3H,t).

### Exemple 3: $\alpha$-carbométhoxy-$\alpha$-méthyltetrahydro-$\beta$-carboline

Une solution de 4,0 g de chlorhydrate de tryptamine (20 mmol) et 20 ml (22 mmol) de pyruvate de méthyle dans 80 ml de méthanol anhydre est portée à reflux pendant 21 heures. Après refroidissement, la solution est concentrée sous vide.

Le résidu solide est dissous dans 40 ml d'eau à chaud, filtré puis ajouté à 3 ml d'une solution concentrée d'hydroxyde d'ammonium. Le précipité cristallin est recristallisé dans un mélange eau:éthanol (3:5) pour fournir ainsi 3,5 g (rendement: 72%) de la $\beta$-carboline II ($R_1$=H, $R_2$=$CH_3$).

Caractéristiques physico-chimiques:

Fusion: 136—138°C (littérature: 138°C)[(1)]

R.M.N. ($CDCl_3$, $\delta$): 8,6 (1H,s); 7,3—7,9 (4H,m); 3,9 (3H,s); 3,3 (2H,t); 2,8 (2H,t).

(1): G. Hahn, D. Scheles, L. Buerwald et H. Werner Just. Lieb. Ann., *520*, 107 (1935).

Exemple 4: (±) vincadifformine (Ia)

Une solution de 300 mg (1,23 mmol) de α-carbométhoxy-α-méthyl-β-carboline (II) et de 0,22 ml (1,5 mmol) de chloro-5 éthyl-2 pentanal et de 1 mg d'acide paratoluène sulfonique dans 25 ml de toluène, est portée à reflux 100 heures sous azote à travers un séparateur d'eau Dean-Stark. On ajoute alors à la solution encore chaude 0,38 ml (3,0 mmol) de diazabicycloundécène (DBU). La solution est encore chauffée pendant 18 heures. Le mélange réactionnel est refroidi, concentré sous vide et le résidu obtenu est dissous dans le chlorure de méthylène. Après filtration à travers une colonne de silice (Baker, 25 g, longueur: 40 cm) et lavage avec une solution de chlorure de méthylène contenant 3% de méthanol, le second éluat de 100 ml est concentré sous vide et fournit 360 mg (84%) de vincadifformine (Ia). Le spectre R.M.N de ce produit correspond à celui d'un échantillon authentique de dl-vincadifformine. Par chromatographie s·· couche mince, on ne détecte que de très faibles quantités d'impuretés. Après recristallisation dans l'acétonitrile, on obtient un échantillon ayant un point de fusion et un point de fusion mixte de 124—125°C (littérature: 124—125°C)[2].

Spectre de masse (80 eV) m/e, intensité relative: 124 (100), 214 (4), 328 (85) $M^+$.

(2): C. Djerassi, H. Budzikiewicz, J. M. Wilson, J. Gosset, M. M. Janot, Tetr. Letters 235 (1962); J. Gosset, J. Le Men, M. M. Janot, Ann. Pharm. France, *20*, 448 (1962).

Exemple 5: carbométhoxy-1-méthoxy-7-méthyl-1-tétrahydro-1,2,3,4, -9H-pyrido (3,4-b) indole (IIb)

On porte à reflux une solution de 113 mg de méthoxy-6-tryptamine[3] et 80 μl de pyruvate de méthyle dans 5 ml de méthanol sous azote pendant 18 heures.

On traite le mélange réactionnel refroidi par 10 ml d'une solution aqueuse saturée en carbonate de sodium et 15 ml de chlorure de méthylène. La phase aqueuse est séparée puis lavée par deux fois 15 ml de chlorure de méthylène. Les phases organiques rassemblées sont lavées avec une solution aqueuse saturée en NaCl, filtrées à travers papier séparateur de phase et concentrées.

Après trituration avec 2 ml d'éther, on obtient 110 mg (rendement: 80%) de produit, fusion 182—184°C.

Un échantillon pour analyse est obtenu par recristallisation dans l'acétate d'éthyle, fusion 184—185°C.

R.M.N. ($CDCl_3$, δ): 8,25 (large s,1H); 7,44 (d,1H); 6,85 (m,2H); 3,84 (s,3H); 3,80 (s,3H); 3,21 (t,2H); 2,72 (t,2H); 2,28 (large s,1H); 1,70 (s,3H).

Analyse élémentaire calculée pour $C_{15}H_{18}N_2O_3$: C=65,67; H=6,61; N=10,21 trouvée: C=65,68; N=9,92; H=6,76.

(3): R. B. Woodward, F. E. Bader, H. Bickel, A. J. Frey and R. W. Kierstead, Tetrahedron 2, 1 (1958).

Exemple 6: ± ervincéine Ib (± méthoxy-16-vincadifformine)

A une solution de 130 mg (0,47 mmol) de l'ester méthoxytétrahydrocarboline IIb et d'un cristal d'acide p-toluène sulfonique dans 3 ml de toluène, on ajoute 100 μl (0,75 mmol) de chloro-5 éthyl-2 pentanal dissous dans 1 ml de toluène. Le mélange réactionnel est reflué durant 72 heures à travers une trappe Dean-Stark contenant du tamis moléculaire 3 A (Davison).

Après concentration et partage du résidu entre 10 ml de HCl à 10% et 5 ml d'hexane, addition d'un excès de KOH aqueux à la phase aqueuse et extraction avec 15 ml de dichlorométhane, une concentration sous vide fournit un résidu basique. Ce dernier est purifié par dissolution dans 50 ml d'acétate d'éthyle et filtration rapide à travers un gel de silice (3 g, Baker). On obtient après concentration de l'éluat 160 mg (92%) d'ervincéine sous forme d'une huile ambrée homogène par C.C.M. (Merck Silica gel, Rf 0,7, acétate d'éthyle). Le produit forme un picrate, point de fusion et point de fusion mixte 183—184°C. Les données spectroscopiques sont identiques à celles d'un échantillon authentique.

R.M.N. ($CDCl_3$ δ): 8,90 (large s,1H); 7,00—7,28 (m,1H); 6,30—6,50 (m,2H); 3,76 (s,6H); 3,40—0,80 (m,15H).

Spectre de masse (80 eV, m/e, intensité relative): 124 (100); 184 (12); 244 (12); 309 (12); 368 (90, $M^+$).

Exemple 7: N(α)-méthyltryptamine

Une solution de 3,20 g (20,0 mmol) de tryptamine et 3,10 g (20,0 mmol) d'anhydride phtalique dans 40 ml de toluène est portée à reflux pendant 12 heures.

Après refroidissement, filtration et concentration, on obtient la phtalimide brute qui est recristallisée dans l'éthanol fournissant ainsi 4,85 g (rendement: 84%) de produit pur. Fusion: 164—165°C (littérature: 164°C)[4].

Une solution de 0,58 g (2,0 mmol) de la phtalimide dans 1,5 ml de dimethylformamide (DMF) est ajoutée en deux minutes à 1 ml de DMF contenant 0,22 mmol d'hydrure de sodium à 50% sous forme de suspension dans l'huile minérale. Après agitation à 20°C pendant 30 minutes et sous azote, on ajoute 0,25 ml (4,0 mmol) d'iodure de méthyle. La solution brun foncé devient jaune pâle. Après 15 minutes, le mélange est versé dans 40 ml d'une solution aqueuse de NaCl à moitié saturée et après 20 minutes, le précipité obtenu est filtré et lavé à l'eau.

Une recristallisation dans l'éthanol du dérivé N-méthyl ainsi obtenu, fournit 0,40 g (65%) de produit pur.

Fusion: 174—175°C (littérature: 175—176°C ou 177—178°C)[5–6].

Un mélange de 824 mg (2,7 mmol) de la N-méthyl tryptamine phtalimide et 0,7 ml (14 mmol) d'hydrate d'hydrazine (solution à 85%, Fisher) dans 80 ml d'éthanol est porté à reflux pendant 24 heures. 20 ml d'une solution aqueuse HCl à 10% sont alors ajoutés et la solution est portée à reflux pendant 30 minutes supplémentaires.

Après refroidissement, concentration et partage du résidu entre 60 ml de chlorure de méthylène et 60 ml d'une solution aqueuse de carbonate de sodium, la phase aqueuse est extraite avec 60 ml de chlorure de méthylène et les phases organiques combinées sont lavées avec une solution aqueuse saturée en NaCl.

Après concentration et distillation (Kugelrohr), on obtient 0,45 g (96%) de l'amine (Eb. 95—105°C à 0,06 mm Hg).

L'huile ainsi obtenue est dissoute dans l'acétate d'éthyle et on laisse barboter du HCl gazeux. Le chlorhydrate d'amine est séparé par filtration et lavé avec de l'acétate d'éthyle contenant du HCl puis avec de l'éther.

La N($\alpha$)-méthyltryptamine sous forme de chlorhydrate a un point de fusion de 201—202°C, littérature 198—199°C[7–8].

(4): R. H. F. Manske, J. Am. Chem. Soc., 51, 1202 (1929).
(5): S. Sugasawa et M. Murayama, Chem. Pharm. Bull., 6, 194 (1958).
(6): T. Hino, ibid, 9, 988 (1961).
(7): H. Wieland, W. Kung et H. Mittash, Justus Liebigs Ann. Chem., 613, 1 (1939).
(8): A. W. Jackson et A. E. Smith, J. Chem. Soc., Suppl. 5510 (1964).

Exemple 8: carbométhoxy-1-méthyl-1-tétrahydro-1,2,3,4-méthyl-9 pyrido (3,4-b) indole (IIc)

160 mg de N($\alpha$)-méthyltryptamine chlorhydrate et 0,1 ml (environ 50% en excès) de pyruvate de méthyle dissous dans 5 ml de méthanol sont portés à reflux sous azote durant 30 heures. La solution refroidie est concentrée sous vide et le résidu est repris par 20 ml d'une solution aqueuse à 10% d'HCl et 10 ml d'hexane. La phase aqueuse est séparée puis rendue basique par addition de KOH et ensuite extraite avec trois fois 20 ml de chlorure de méthylène.

Les phases organiques réunies sont lavées avec NaCl aqueux saturé, concentrées à sec et le résidu, dissous dans l'acétate d'éthyle, est filtré à travers 4 g de gel de silice Baker.

Après concentration puis distillation boule à boule, (Eb. 150°C—160°C, 0,03 mmHg), on obtient 158 mg (80%) de tétrahydro-$\beta$-carboline.

R.M.N. ($CDCl_3$, $\delta$): 7,3 (m,4H); 3,77 (s,3H); 3,70 (s,3H); 3,18 (t,2H); 2,78 (t,2H); 2,22 (s large, 1H); 1,75 (s,3H).

Analyse élémentaire: calculé pour $C_{15}H_{18}N_2O_2$: C = 69,74; H = 7,02; N = 10,85; trouvé: C = 69,50; H = 6,94; N = 10,63.

Point de fusion du picrate recristallisé dans le méthanol: 190—191°C.

Exemple 9

Minovine (I, $R_1$=$R_2$=H; $R_3$=méthyle, $R_4$=éthyle, $R_5$=méthoxy)

Le mode opératoire est similaire à celui suivi pour l'obtention de l'ervincéine.

A partir de 129 mg de $\beta$-carboline (IIc) et en utilisant 50 ml d'éther au lieu d'acétate d'éthyle dans l'étape de filtration à travers 2 g de gel de silice, on obtient 140 mg d'un résidu brut contenant deux produits par C.C.M. (Rf 0,2 et 0,7 — majeur: minovine —, acétate d'éthyle, silica gel). Par chromatographie sur couche mince préparative, on isole 65 mg (37%) de minovine sous forme d'huile dont le spectre de R.M.N. est identique à celui d'un échantillon obtenu par méthylation de la ± vincadifformine.

L'échantillon obtenu par synthèse totale cristallise lentement dans un mélange hexane:éther (10:1) et a un point de fusion et un point de fusion mixte de 119—121°C.

Le picrate est recristallisé dans le méthanol:éther, fusion 194—197°C.

Des expériences subséquentes, réalisées à un échelle plus importante, ont permis d'isoler la minovine avec un rendement total de 56% sans séparation chromatographique.

Préparation de l'échantillon de référence:

Une solution de 34 mg (0,1 mmol) de ± vincadifformine dans 1 ml de DMF est additionnée à température ambiante à un mélange de 10 mg (0,2 mmol) de NaH à 50% dans l'huile minérale, dissous dans 1 ml de DMF.

Après 10 minutes, l'addition de 5 ml d'eau provoque la précipitation d'un produit sirupeux. Après décantation et séparation du solvant, addition d'éther puis filtration de cette solution d'éther à travers un papier séparateur de phase, et enfin, concentration sous vide, on obtient 30 mg (85%) de minovine.

Par R.M.N., on n'observe plus de NH indolique à $\delta$ 8,9 mais le spectre présente une absorption correspondant au singulet N—$CH_3$ à 3,24 et dont l'intégration correspond à trois protons.

Le picrate fond à 194—197°C et la base libre régénérée à 119—121°C, littérature 120—122°C[9].

Spectre de masse (80 eV, m/e, intensité relative: 124 (100) 168 (7); 228 (4); 267 (7); 352 (55) $M^+$.

(9): J. Mokry, I. Kompis, L. Dubavkova et P. Sefcovic, Experientia, 19, 311 (1963).

Exemple 10

a) acétyl-1 méthyl-1 tétrahydro-1,2,3,4, 9H-pyrido(3,4-b)indole

10,01 g de chlorhydrate de tryptamine (50,7 mmol) et 4,8 g de butanedione sont mis en solution dans 150 ml de méthanol. La solution est portée à reflux pendant 20 heures. On concentre sous vide et lave le résidu à l'éther. On ajoute alors 50 ml d'une solution aqueuse 10% ammoniacale et on extrait au chlorure de méthylène. La phase organique est séchée sur $MgSO_4$, filtrée puis concentrée sous vide. On obtient ainsi une huile qui cristallise dans un mélange $CH_3OH/H_2O$ pour fournir 8,5 g de carboline (rendement: 71%). Fusion: 83,2°C.

I.R. ($CHCl_3$, $cm^{-1}$): 3450, 2929, 1705, 1603, 1460.

R.M.N. ($CDCl_3$, $\delta$): 8,76 (slarge, 1H); 7,00—7,63 (m,4H); 2,60—3,53 (m,4H); 2,33 (s,3H); 1,85 (s,1H); 1,53 (s,3H).

b) acétyl-16 aspidospermidine (I, $R_1$=$R_2$=$R_3$=H, $R_4$=éthyle, $R_5$=méthyle)

Dans un ballon muni d'un séparateur d'eau Dean-Stark et mis sous atmosphère d'argon, on introduit 5,63 g (24,7 mmol) de la carboline obtenue en a), 10,4 g (50 mmol) de sulfométhoxy-5 éthyl-2 pentanal, 110 mg d'acide p-toluène sulfonique et 400 ml d'un mélange benzène:toluène (3:7). La solution ainsi obtenue est portée à reflux pendant 100 heures. On ajoute alors à la solution encore chaude 7,5 ml de DBU. La solution noircit instantanément et on continue à chauffer pendant 24 heures. Après refroidissement, on décante la solution et concentre sous vide.

On obtient ainsi 11,5 g d'une huile. On sépare et purifie l'acétyl-16 aspidospermidine par chromatographie sur colonne de silice (300 g de $SiO_2$, éluant $CH_2Cl_2$:$CH_2OH$ 97:3). On obtient ainsi 4,21 g d'acétyl-16 aspidospermidine (rendement: 42%).

R.M.N. ($CDCl_3$, $\delta$): 10,73 (large s,1H); 6,70—7,43 (m,4H); 0,80—3,40 (m,15H); 2,26 (s,3H); 0,63 (t,3H).

I.R. (film, $cm^{-1}$): 3310, 2930, 2770, 1645, 1620, 1550, 1460, 1250, 1155.

**Revendications**

1. Procédé de préparation de la vincadifformine et d'autres alcaloïdes pentacycliques similaires de formule générale

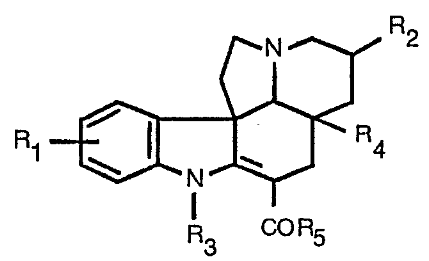

dans laquelle:

$R_1$ représente un atome d'hydrogène ou d'halogène, un groupe hydroxy ou un groupe acyloxy, alkoxy ou alkyle comportant de 1 à 4 atomes de carbone,

$R_2$ représente un atome d'hydrogène ou un groupe hydroxyle,

$R_3$ représente un atome d'hydrogène ou un groupe alkyle comportant de 1 à 4 atomes de carbone,

$R_4$ représente un groupe alkyle, éventuellement substitué par un radical hydroxy ou alkoxy, comportant de 1 à 7 atomes de carbone,

$R_5$ est un groupe méthyle ou un groupe alkoxy comportant de 1 à 4 atomes de carbone, caractérisé en ce que l'on procède à une condensation de la carboline répondant à la formule générale

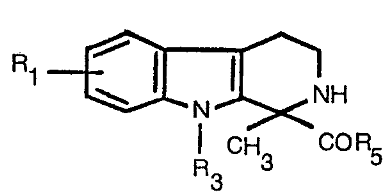

dans laquelle $R_1$, $R_3$ et $R_5$ ont les significations mentionnées ci-dessus, avec un aldéhyde de formule générale

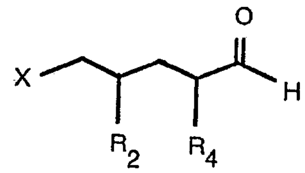

dans laquelle $R_2$ et $R_4$ ont les significations mentionnées ci-dessus et X représente un bon groupe partant tel un halogène, un groupe arylsulfonyloxy, alkylsulfonyloxy, ou fluoroalkylsulfonyloxy, cette condensation étant effectuée dans un solvant inerte dans les conditions de réactions et l'aldéhyde utilisé pouvant éventuellement se présenter sous une forme acétalisée ou hémiacétalisée.

2. Le procédé selon la revendication 1 caractérisé en ce que l'aldéhyde utilisé est le chloro-5 éthyl-2 pentanal.

3. Le procédé selon la revendication 1 caractérisé en ce que la condensation est effectuée en présence d'un catalyseur acide.

4. Le procédé selon la revendication 3 caractérisé en ce que le catalyseur acide est l'acide p-toluènesulfonique.

5. Le procédé selon la revendication 1 caractérisé en ce que la condensation est effectuée en deux stades, le premier étant le chauffage du milieu réactionnel en présence d'un catalyseur acide et le second stade étant le chauffage après l'addition d'une base non nucléophile.

6. Le procédé selon la revendication 5 dans laquelle la base est le diazabicycloundécène.

7. Le procédé selon la revendication 1 caractérisé en ce que la condensation est effectuée dans le benzène.

8. Le procédé de la revendication 1 caractérisé en ce que la condensation est effectuée dans le toluène.

**Patentansprüche**

1. Verfahren zur Herstellung des Vincadifformins und anderer ähnlicher pentacyclischer Alkaloide der allgemeinen Formel

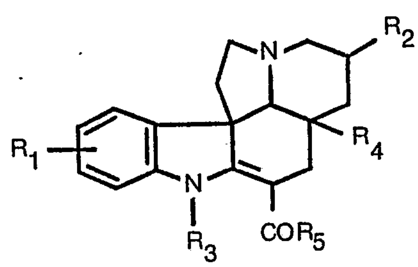

worin

$R_1$ ein Wasserstoff- oder Halogenatom, eine Hydroxygruppe oder eine 1 bis 4 Kohlenstoffatome enthaltende Acyloxy-, Alkoxy- oder Alkylgruppe bedeutet,

$R_2$ ein Wasserstoffatom oder eine Hydroxylgruppe bedeutet,

$R_3$ ein Wasserstoffatom oder eine 1 bis 4 Kohlenstoffatome enthaltende Alkylgruppe bedeutet,

$R_4$ eine 1 bis 7 Kohlenstoffatome enthaltende Alkylgruppe, die gegebenenfalls durch einen Hydroxy- oder Alkoxyrest substituiert ist, bedeutet, und

$R_5$ eine Methylgruppe oder eine 1 bis 4 Kohlenstoffatome enthaltende Alkoxygruppe ist, dadurch gekennzeichnet, daß man eine Kondensation eines Carbolins der allgemeinen Formel

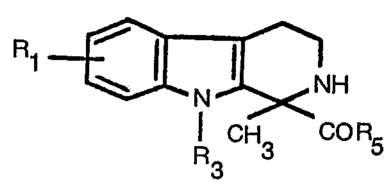

worin $R_1$, $R_3$ und $R_5$ die oben angegebenen Bedeutungen haben, mit einem Aldehyd der allgemeinen Formel

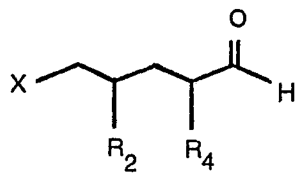

worin $R_2$ und $R_4$ die oben angegebenen Bedeutungen haben und X eine geeignete austretende Gruppe, wie ein Halogen, eine Arylsulfonyloxy-, Alkylsulfonyloxy- oder Fluoralkylsulfonyloxygruppe bedeutet, vornimmt, diese Kondensation in einem unter den Reaktionsbedingungen inerten Lösungsmittel bewirkt und den verwendeten Aldehyd gegebenenfalls in acetalisierter oder hemiacetalisierter Form einsetzt.

2. Das Verfahren nach Anspruch 1, dadurch gekennzeichnet, daß der verwendete Aldehyd Chlor-5-ethyl-2-pentanal ist.

3. Das Verfahren nach Anspruch 1, dadurch gekennzeichnet, daß die Kondensation in Gegenwart eines sauren Katalysators bewirkt wird.

4. Das Verfahren nach Anspruch 3, dadurch gekennzeichnet, daß der saure Katalysator p-Toluolsulfonsäure ist.

5. Das Verfahren nach Anspruch 1, dadurch gekennzeichnet, daß die Kondensation zweistufig vorgenommen wird, wobei in der ersten Stufe das Reaktionsmilieu in Gegenwart eines sauren Katalysators erhitzt wird und in der zweiten Stufe das Erhitzen nach Zugabe einer nicht-nukleophilen Base erfolgt.

6. Das Verfahren nach Anspruch 5, in welchem die Base Diazabicycloundecen ist.

7. Das Verfahren nach Anspruch 1, dadurch gekennzeichnet, daß die Kondensation in Benzol bewirkt wird.

8. Das Verfahren des Anspruchs 1, dadurch gekennzeichnet, daß die Kondensation in Toluol bewirkt wird.

## Claims

1. A process for preparing vincadifformine and other related pentacyclic alcaloids of the general formula

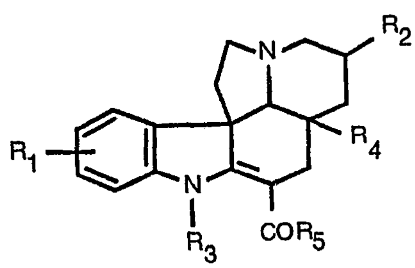

wherein

$R_1$ is hydrogen or halogen, an hydroxy group or an acyloxy, alkoxy or alkyl group having from 1 to 4 carbon atoms,

$R_2$ is hydrogen or a hydroxyl group,

$R_3$ is hydrogen or an alkyl group having from 1 to 4 carbon atoms,

$R_4$ is an alkyl group, possibly substituted by a hydroxyl or alkoxy substituent, having from 1 to 7 carbon atoms,

$R_5$ is a methyl group or an alkoxy group having from 1 to 4 carbon atoms,
characterized in that there is condensed a carboline having the general formula

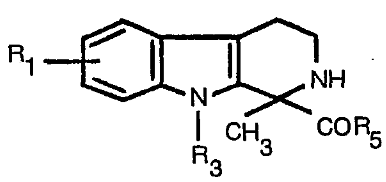

wherein $R_1$, $R_3$, and $R_5$ have the abovementioned meanings, with an aldehyde of the general formula

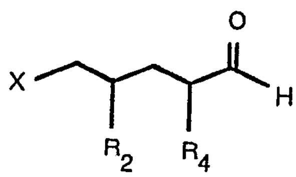

wherein $R_2$ and $R_4$ have the abovementioned meanings and X is a good leaving group such as a halogen, an arylsulfonyloxy, alkylsulfonyloxy or fluoroalkylsulfonyloxy group,
such condensation being performed in an inert solvent in the reaction conditions and the aldehyde which is used being possibly in the acetalised or hemiacetalised form.

2. The process of claim 1 characterized in that the aldehyde used is 5-chloro-2-ethyl-pentanal.

3. The process of claim 1 characterized in that the condensation is performed in the presence of an acid catalyst.

4. The process of claim 3 characterized in that the acid catalyst is p-toluenesulfonic acid.

5. The process of claim 1 characterized in that the condensation is performed in two steps, the first one being heating of the reaction mixture in the presence of an acid catalyst and the second step being heating after the addition of a non-nucleophilic base.

6. The process of claim 5 wherein the base is diazabicycloundecene.

7. The process of claim 1 characterized in that the condensation is performed in benzene.

8. The process of claim 1 characterized in that the condensation is performed in toluene.